# EUROPEAN PATENT SPECIFICATION

(11) **EP 1 857 107 B1**
(45) Date of publication and mention of the grant of the patent: **06.04.2011**
(21) Application number: 06714196.0
(22) Date of filing: 21.02.2006
(51) Int. Cl.: A61K 31/4188, C07D 491/107, A61P 9/00

(54) **PREVENTIVE OR THERAPEUTIC AGENT FOR CARDIAC DYSFUNCTION OR MYOCARDIAL DAMAGE CAUSED BY ISCHEMIA OR ISCHEMIA-REPERFUSION**
MITTEL ZUR PRÄVENTION ODER BEHANDLUNG VON HERZDYSFUNKTION ODER MYOKARDSCHÄDEN DURCH ISCHÄMIE ODER ISCHÄMIE-REPERFUSION
AGENT PRÉVENTIF OU THÉRAPEUTIQUE POUR DYSFONCTION CARDIAQUE OU DOMMAGE MYOCARDIQUE PROVOQUÉ PAR ISCHÉMIE OU REPERFUSION D ISCHÉMIE

(30) Priority: 22.02.2005 JP 2005044889
(43) Date of publication of application: 21.11.2007
(73) Proprietor: Sanwa Kagaku Kenkyusho Co., Ltd, Nagoya-shi Aichi 461-8631 (JP); Yabe, Chihiro, Kyoto-shi, Kyoto, 606-0003 (JP)
(72) Inventor: IWATA, Kazumi, akyo-ku, Kyoto-shi, Kyoto, 6060024 (JP); YABE, Chihiro, Kyoto-shi, Kyoto 606 0003 (JP)
(74) Representative: Chapman, Helga Claire
(86) International application number: PCT/JP2006/303056
(87) International publication number: WO 2006/090699

(56) References cited:
- EP-A1- 1 064 969
- JP-A- 63 057 588
- JP-A- 2002 504 884
- YOREK M A ET AL: "Effect of fidarestat and alpha-lipoic acid on diabetes-induced epineurial arteriole vascular dysfunction." EXPERIMENTAL DIABESITY RESEARCH 2004 APR-JUN, vol. 5, no. 2, April 2004 (2004-04), pages 123-135, XP002528258 ISSN: 1543-8600
- ASANO TOMOICHIRO ET AL: "Fidarestat (SNK-860), a potent aldose reductase inhibitor, normalizes the elevated sorbitol accumulation in erythrocytes of diabetic patients." JOURNAL OF DIABETES AND ITS COMPLICATIONS 2002 MAR-APR, vol. 16, no. 2, March 2002 (2002-03), pages 133-138, XP002528259 ISSN: 1056-8727
- TRACEY W R ET AL: "Aldose reductase inhibition alone or combined with an adenosine A(3) agonist reduces ischemic myocardial injury." AMERICAN JOURNAL OF PHYSIOLOGY. HEART AND CIRCULATORY PHYSIOLOGY OCT 2000, vol. 279, no. 4, October 2000 (2000-10), pages H1447-H1452, XP002528260 ISSN: 0363-6135
- HWANG YUYING C ET AL: "Central role for aldose reductase pathway in myocardial ischemic injury." THE FASEB JOURNAL : OFFICIAL PUBLICATION OF THE FEDERATION OF AMERICAN SOCIETIES FOR EXPERIMENTAL BIOLOGY AUG 2004, vol. 18, no. 11, August 2004 (2004-08), pages 1192-1199, XP002528261 ISSN: 1530-6860
- HWANG Y.C. ET AL.: 'Aldose reductase activation is a key component of myocardial response to ischemia' THE FASEB LETTERS vol. 16, February 2002, pages 243 - 245, XP002268367

## Description

### BACKGROUND

Diseases induced by ischemia or ischemia reperfusion, for example a cardiac event or cardiac death caused by an acute coronary syndrome such as myocardial infarction and angina, are known and have a poor prognosis in many cases. Drug treatment for these diseases is limited to blood pressure management and hemodynamic control or maintenance such as thrombolysis. There is no fundamental therapeutic agent for protecting a heart or myocardium.

Coronary artery disease among Japanese has increased in number year after year, and the number of operation cases for ischemic heart disease in Japan exceeded 20000 cases in the year 2000. As methods of coronary revascularization for an ischemic heart disease, percutaneous transluminal coronary angioplasty (PTCA), which is an internal therapy, and coronary artery bypass grafting (CABG) by surgical operation are performed. Thus, preventing development into myocardial infarction and sudden cardiac death, and to improve vital prognosis, is important. Reperfusion therapy by PTCA is said to be effective, but restenosis or re-infarction is manifested at an early stage in many cases. When the disease state has led to end stage ischemic cardiomyopathy, and heart transplantation is applied, application of transplantation is extremely difficult, and lack of a donor in heart transplantation is a serious limitation on a global scale.

As current drug therapy to prevent coronary artery stenosis or obstruction, administration of an antihypertensive agent, such as a calcium blocker or an angiotensin receptor blocker, or of a anti-hyperlipidemic agent such as an HMG-CoA reductase inhibitor, which is a therapeutic agent for hypertension, hyperlipidemia or arteriosclerosis that causes ischemic heart disease, has been tried. However, these drug therapies are not reliably effective currently. In addition, when the disease state has led to heart failure, a cardiotonic agent is said to be effective, but it is said that the workload of myocardium is increased and the effect is temporary. Therefore, there is no effective therapeutic agent when acute myocardial infarction or arrhythmia has led to acute coronary syndrome, and intervention by a drug treatment for protecting myocardium is necessary in addition to execution of early stage reperfusion. However, in execution of early stage reperfusion, in case that reperfusion is performed at a time when ischemia lasts and injury of cardiomyocytes becomes apparent, injury of myocardium is not restored, but is exacerbated in some cases (reperfusion myocardial injury). For this reason, the problem of how to prevent ischemia or ischemia reperfusion injury is of considerable interest. As a pathogenesis for development of the myocardial ischemia or ischemia reperfusion injury, an intracellular ATP deficiency theory, a Ca overloading theory and a free radical theory have previously been widely proposed.

Further, in valve replacement or valveplasty for a heart valve disease, or in treatment of a congenital cardiac abnormality (ventricular septal defect, atrial septal defect, lung artery stenosis etc.), open heart surgery using extracorporeal circulation under an artificial heart and lung is performed. Since cardiac muscle is in an oxygen deficient state during such open heart surgery, operation time is restricted to prevent myocardial necrosis. Further, since severe arrhythmia or reduction in force of cardiac contraction (heart failure state) is developed by open heart surgery loading or reperfusion injury after open heart surgery (ischemia reperfusion injury), administration of an anti-arrhythmia agent or a cardiotonic agent is necessary, and the necessity of strict management in an intensive care unit has become a big problem in open heart surgery.

Recently, there has been a report that zopolrestat, an aldose reductase (AR) inhibitor, improves the cardiac performance of diabetic cardiomyopathy patients (Johnson BF: Diabetes Care 27,448,2004). However, subsequent drug development of zopolrestat has ceased due to manifestation of side effects such as hepatic disorders.

On the other hand, (2S,4S)-6-fluoro-2',5'-dioxospiro[chroman-4,4'-imidazolidin e]-2-carboxamide (general name: fidarestat, development code: SNK-860), a compound discovered by the present applicant company, was developed as a compound having a strong aldose reductase inhibitory activity but having high safety, even when taken over a long period. Currently, a clinical trial is in progress worldwide to test fidarestat as a therapeutic agent for diabetic neuropathy.

Regarding fidarestat, its use in diabetic neuropathy is described in Japanese Patent Application Laid-Open (JP-A) No.61-200991, its use in various diseases accompanied with aging is described in JP-A No.6-135968, its use in diabetic simple retinopathy is described in JP-A No.7-242547, and its use in diabetic keratopathy is described in JP-A No.8-231549. In addition, regarding a hydantoin derivative having a similar structure, use in circulation diseases is described in JP-A No.4-173791, but as reported in Journal of Technical Disclosure 2006-500058, fidarestat has no such pharmacological effects.

### SUMMARY OF THE INVENTION

As described above, establishment of therapy for cardiac dysfunction or myocardial injury caused by ischemia or ischemia reperfusion having high effectiveness and safety is strongly sought by the medical field. Particularly, from the viewpoint of safety of internal therapy and surgical operation therapy, the appearance of a therapeutic agent with high safety which can be taken over a long period is currently strongly desirable. Thus, it is beneficial to provide a preventive or therapeutic agent for cardiac dysfunction or myocardial injury caused by ischemia or ischemia reperfusion which exhibits effectiveness by a different mechanism from that of the existing therapeutics and which can be taken over a long period.

The present inventors assessed (2S,4S)-6-fluoro-2',5'-dioxospiro[chroman-4,4'-imidazolidin e]-2-carboxamide (general name: fidarestat) using a heart ischemia-reperfusion disorder model which is generally used. As a result, it was found out that the drug is effective for cardiac dysfunction or myocardial injury caused by ischemia or ischemia reperfusion. That is, the present invention may comprise a preventive or therapeutic agent for cardiac dysfunction or myocardial injury caused by ischemia or ischemia reperfusion, containing 6-fluoro-2',5'-dioxospiro[chroman-4,4'-imidazolidine]-2-car boxamide (including the racemate) as an active ingredient.

The cardiac dysfunction or myocardial injury caused by ischemia or ischemia reperfusion is ischemia or an ischemia reperfusion injury in the heart. Specifically, some examples may include reperfusion arrhythmia, a cardiac event or cardiac death. These are classified into those caused by acute coronary syndrome such as unstable angina and myocardiac infarction, those caused by percutaneous transluminal coronary angioplasty (PTCA) for therapy thereof, those caused by an ischemia reperfusion injury of myocardium inextracorporeal circulation under an artificial heart and lung, and those caused by open heart surgery such as coronary artery bypass grafting operation without using an artificial heart and lung.

The preventive or therapeutic agent for cardiac dysfunction or myocardiac injury caused by ischemia or ischemia reperfusion described herewith may have a characteristic that it exhibits the remarkable effect at a lower dose as compared with other AR inhibitors, and has no problem from the viewpoint of safety. That is, the claims herein may encompass, among other things, providing a preventive or therapeutic agent for cardiac dysfunction or myocardiac injury cased by ischemia or ischemia reperfusion, which can be administered for a long period.

### BRIEF DESCRIPTION OF THE DRAWINGS

Embodiments will now be described, by way of example only, with reference to the accompanying drawings which are meant to be exemplary and wherein like elements are numbered alike in several Figures, in which:
Fig. 1 shows an experimental protocol.
Fig. 2 shows the effect of AR inhibitors on increase in LVEDP in wild type mice.
Fig. 3 shows the effect of AR inhibitors on reduction in +dP/dt max in wild type mice.
Fig. 4 shows the effect of AR inhibitors on the release of CK from cardiomyocytes in wild type mice.
Fig. 5 shows the effect of AR inhibitors on increase in LVEDP in hAR-TG mice.
Fig. 6 shows the effect of AR inhibitors on reduction in +dP/dt max in hAR-TG mice.
Fig. 7 shows the effect of AR inhibitors on the release of CK from cardiomyocytes in hAR-TG mice.
Fig. 8 shows the effect of AR inhibitors on the myocardial ATP content in hAR-TG mice.

### DETAILED DESCRIPTION

The present compound can be orally administered for example, as tablets, capsules, powders, granules, liquids or syrups, or can be parenterally administered as injectables or suppositories, which were formed by conventional pharmaceutical manufacturing techniques. For pharmaceutical manufacturing, in the case of solid formulations, pharmaceutically acceptable excipients such as starch, lactose, purified white sugar, glucose, crystalline cellulose, carboxycellulose, carboxymethylcellulose, carboxyethylcellolose, calcium phosphate, magnesium stearate, gum arabic and the like can be used and, if necessary, lubricants, binders, disintegrating agents, coating agents, coloring agents and the like can be incorporated. In addition, in the case of liquid formulations, stabilizers, solubilizers, suspending agents, emulsifiers, buffers, and preservatives can be used. The dose is different depending on symptoms, age, administration methods, dosage forms and the like and, in the normal case, it is preferable that the preparation is administered to an adult in a range of 1 to 200mg, preferably 1 to 100mg per day in terms of the present compound for consecutive days, once or a few times a day.

Embodiments will now be described, by way of example only, and are meant to be exemplary.

### Examples

In the present invention, not only wild-type mice, but also human aldose reductase (AR) transgenic mice (hAR-TG) which was produced so as to overexpress human-type AR genetically in a mouse, were used for predicting effectiveness in humans which is significant for the development of such a therapeutic agent. As a control for comparison with SNK-860, epalrestat and zopolrestat were used.

### 1. Material and methods of pharmacological test

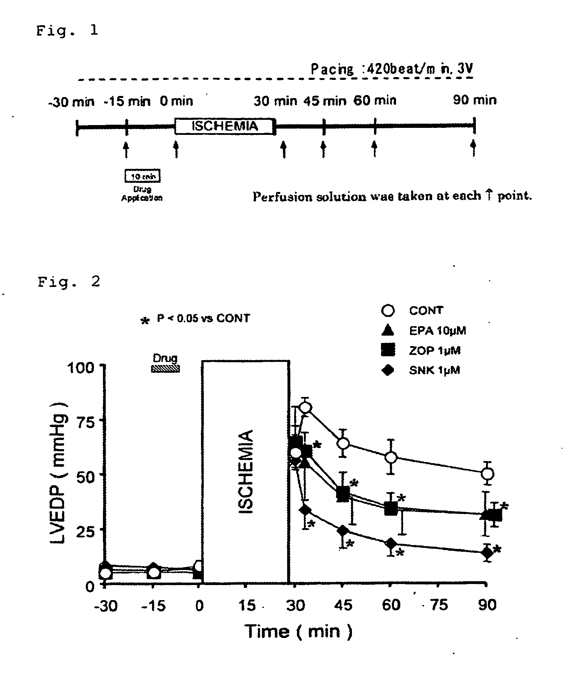

In an experimental test, hearts of 7 to 9 week-old wild-type mice (BDF-1) and hAR-TG were used. From these mice, hearts were isolated under anesthesia of pentobarbital 50mg/kg i.p., and were placed in ice-cold saline. The isolated hearts were perfused for 20 minutes at a perfusion pressure of 70mmHg with the Langendorff apparatus (Model IH-1 Type 844, HUGO SACHS ELEKTRONIK, Germany), and was stabilized. Thereafter, after perfusion for 30 minutes under heart function measurement, perfusion solution was completely stopped for 30 minutes, and reperfusion was performed for further 60 minutes, thereby, ischemia-ischemia reperfusion loading was performed. As perfusion solution, Krebs-Henseleit (KH) buffer containing 5.55 mM glucose and 2 mM Na-pyruvate was used. Cardiac performance was assessed by a left ventricular end-diastolic pressure (LVEDP) and peak first derivative of left ventricular systolic pressure(+dP/dt max). These were measured with a pressure transducer connected with a balloon inserted into a left ventricle in condition of hearts paced at 3 volt, 420 beats/min with an electrode placed on the top of a right ventricle, and data thereof were calculated with a four cannel recording device (OMUNIACE RT-3300, NEC, Japan). An AR inhibitor was added to the perfusion solution for 10 minutes from 15 minutes before global ischemia initiation as shown in Fig. 1. Each inhibitor (1 µM SNK-860: SNK, 1-10 µM zopolrestat: ZOP, 10 µM epalrestat: EPA) was dissolved in DMSO, and a DMSO final concentration in a perfusion solution was adjusted to 0.05%. DMSO of the same concentration was also added to a perfusion solution of a control experiment. Cardiomyocytes injury was determined using as an index the release of total creatinine kinase(CK) for 60-min reperfusion, and a myocardial ATP content after 60 minutes reperfusion was determined by the bioluminescence method (Sigma-Aldrich, St.Louis, MO) using luciferase, respectively.

For hAR-TG mice, littermates (LM: hAR non-expression litter mouse) were used as control mice without drug treatment.

### 2. Results

### (i) Effects on wild-type mice

In the experimental study using wild-type mice, a remarkable increase of the left ventricular end-diastolic pressure (LVEDP) in control isolated heart recognized after ischemia-ischemia reperfusion was significantly improved in an isolated heart of the 1 µM SNK-860 or 1 µM zopolrestat-addition group (Fig. 2).

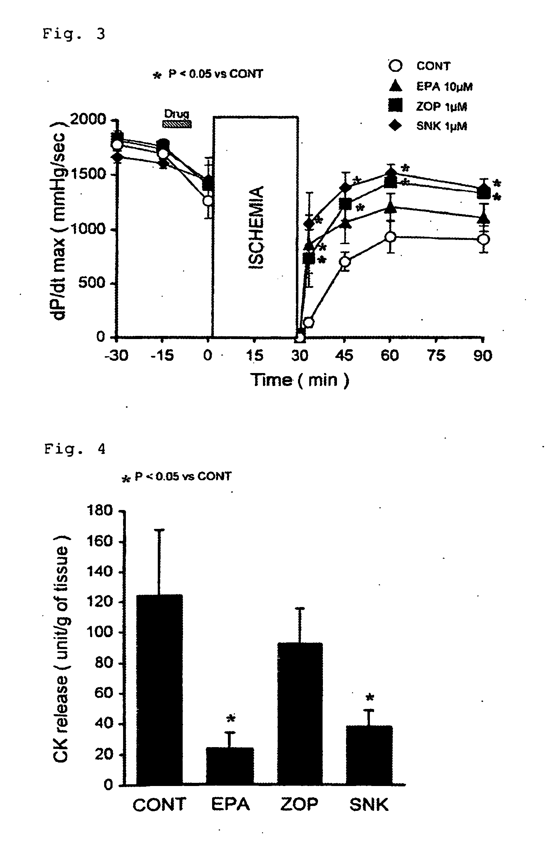

Reduction in peak first derivative of left ventricular systolic pressure (+dP/dt max) immediately after reperfusion was significantly improved in any of the addition groups of three kinds of AR inhibitors, and the most remarkable effect was observed in the 1 µM SNK-860-addition group (Fig. 3).

Regarding the release of CK from cardiomyocytes, a significant reduction was observed in the 10 µM epalrestat and 1 µM SNK-860-addition groups, but no effect was observed in the 1 µM zopolrestat-addition group (Fig. 4).

### (ii) Effects on hAR-TG mice

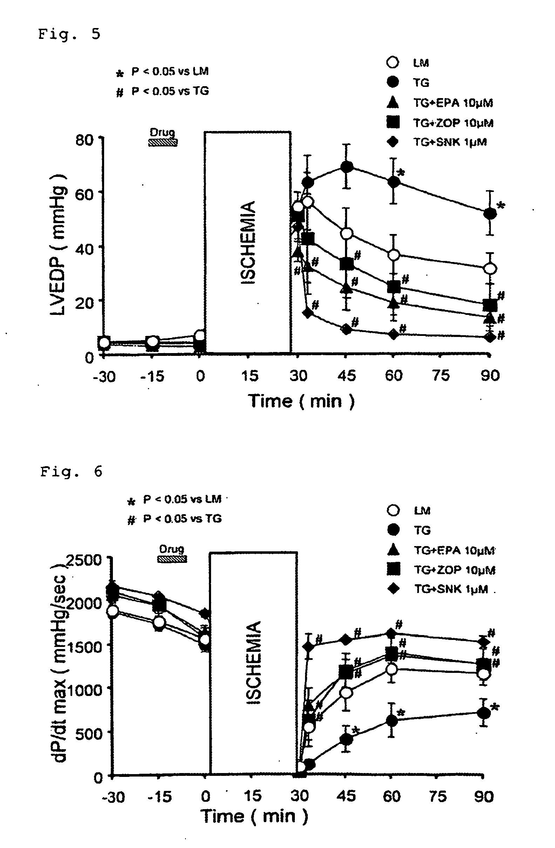

As shown in Figs. 5 to 8, in hAR-TG (TG) mice, reduction in the cardiac performance observed at reperfusion (increase in LVEDP, reduction in +dP/dt max), release of CK into a perfusion solution, and decrease in myocardial ATP content were significantly exacerbated, respectively, as compared with LM. The AR activity in a heart of hAR-TG showed an activity which was about 1.7-fold of that of LM.

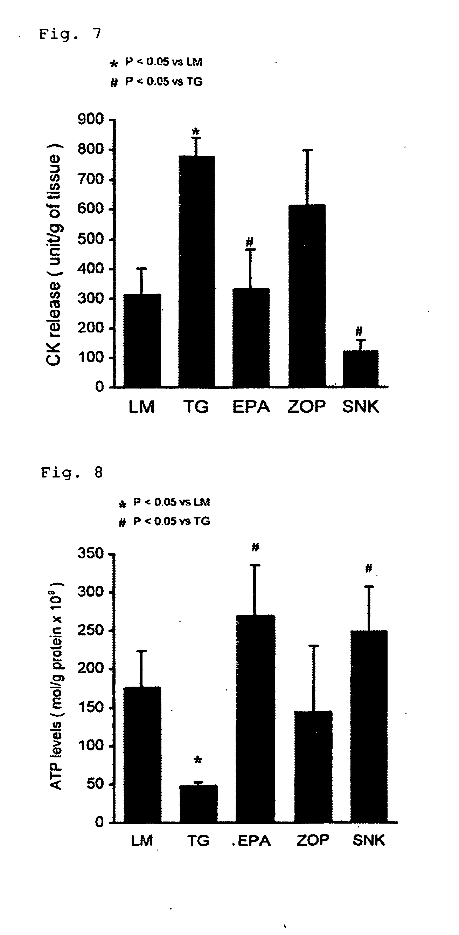

In hAR-TG (TG), regarding increase in LVEDP and reduction in +dP/dt max, significant improvement was observed in the 1 µM SNK-860, 10 µM zopolrestat, or 10 µM mepalrestat-addition group (Fig. 5, 6). On the other hand, regarding release of CK into a perfusion solution, and decrease in a myocardial ATP content, significant improvement was not observed in the 10 µm zopolrestat-addition group, while in the 1 µM SNK-860-addition group, equivalent improvement effect to that of the 10 µM epalrestat-addition group was observed (Fig. 7, 8).

### 3. Discussion

These results show that SNK-860 completely inhibited deterioration of the cardiac performance and destruction of cardiac muscle due to an ischemia reperfusion injury in a heart also in non-diabetic state. In addition, though only the partial effect was seen at a high concentration in other AR inhibitors such as zopolrestat and epalrestat, SNK-860 showed the perfect effect at a lower concentration, and was extremely excellent in respect of action intensity.

The presently disclosed embodiments are therefore to be considered in all respects as illustrative and not restrictive, the scope of the invention being indicated by the appended claims.

## Claims

1. A preventive or therapeutic agent for use in cardiac dysfunction or myocardial injury caused by ischemia or ischemia reperfusion, containing 6-fluoro-2',5'-dioxospiro[chroman-4,4'-imidazolidine]-2-car boxamide as an active ingredient.

2. The preventive or therapeutic agent for the use according to claim 1, wherein the cardiac dysfunction or myocardial injury caused by ischemia or ischemia reperfusion is reperfusion arrhythmia, cardiac event or cardiac death.

3. The preventive or therapeutic agent for the use according to claim 1, wherein the cardiac dysfunction or myocardial injury caused by ischemia or ischemia perfusion is the one caused by acute coronary syndrome such as unstable angina and myocardial infarction, or the one caused by percutaneous transluminal coronary angioplasty (PTCA) for therapy thereof.

4. The preventive or therapeutic agent for the use according to claim 1, wherein the cardiac dysfunction or myocardial injury caused by ischemia or ischemia perfusion is the one caused by ischemia reperfusion injury of myocardium in extracorporeal circulation under an artificial heart and lung, or the one caused by open heart surgery such as coronary artery bypass grafting operation without using an artificial heart and lung.

5. The preventive or therapeutic agent for the use according to any one of claims 1 to 4, wherein the compound is (2S,4S)-6-fluoro-2',5'-dioxospiro[chroman-4,4'-imidazolidine]-2-car boxamide (general name; fidarestat).

## Patentansprüche

1. Vorbeugendes oder therapeutisches Mittel zur Verwendung bei einer durch Ischämie oder Ischämie-Reperfusion verursachten Herzfunktionsstörung oder Myokardverletzung, das 6-Fluor-2', 5'-dioxospiro[chroman-4,4'-imidazolidin]-2-carboxamid als Wirkstoff enthält.

2. Vorbeugendes oder therapeutisches Mittel zur Verwendung gemäß Anspruch 1, wobei die Herzfunktionsstörung oder Myokardverletzung, die durch Ischämie oder Ischämie-Reperfusion verursacht wurde, Reperfusionsarrhythmie, ein Herzereignis oder Herztod ist.

3. Vorbeugendes oder therapeutisches Mittel zur Verwendung gemäß Anspruch 1, wobei die durch Ischämie oder Ischämie-Perfusion verursachte Herzfunktionsstörung oder Myokardverletzung eine solche ist, die durch ein akutes Koronarsyndrom wie instabile Angina und Myokardinfarkt verursacht wird, oder eine solche, die durch perkutane transluminale koronare Angioplastie (PTCA) für deren Therapie verursacht wird.

4. Vorbeugendes oder therapeutisches Mittel zur Verwendung gemäß Anspruch 1, wobei die durch Ischämie oder Ischämie-Perfusion verursachte Herzfunktionsstörung oder Myokardverletzung eine solche ist, die durch Ischämie-Reperfusionsverletzung des Herzmuskels im extrakorporalen Kreislauf unter Verwendung einer Herz-Lungen-Maschine verursacht wird, oder eine solche, die durch eine Operation am offenen Herzen wie z. B. eine Koronararterienbypassoperation ohne Verwendung einer Herz-Lungen-Maschine verursacht wird.

5. Vorbeugendes oder therapeutisches Mittel zur Verwendung nach einem der Ansprüche 1 bis 4, wobei die Verbindung (2S,4S)-6-Fluor-2',5'-dioxospiro[chroman-4,4'-imidazolidin)-2-carboxamid (allgemeiner Name: Fidarestat) ist.

## Revendications

1. Agent prophylactique ou thérapeutique pour utilisation pour des troubles cardiaques ou des lésions du myocarde causés par une ischémie ou une ischémie-reperfusion, contenant du 6-fluoro-2',5'dioxospiro[chroman-4,4'-imidazolidine]-2-carboxamide en tant que principe actif.

2. Agent prophylactique ou thérapeutique pour utilisation selon la revendication 1, le trouble cardiaque ou la lésion myocardique causés par une ischémie ou une ischémie-reperfusion étant une arythmie de reperfusion, un événement cardiaque ou une mort cardiaque.

3. Agent prophylactique ou thérapeutique pour utilisation selon la revendication 1, le trouble cardiaque ou la lésion myocardique causés par une ischémie ou une ischémie-reperfusion étant celui ou celle causé par un syndrome coronaire aigu tel qu'un angor instable ou un infarctus du myocarde, ou celui ou celle causé par une angioplastie coronarienne transluminale percutanée (ACTP) pour traitement afférent.

4. Agent prophylactique ou thérapeutique pour utilisation selon la revendication 1, le trouble cardiaque ou la lésion myocardique causés par une ischémie ou une ischémie-reperfusion étant celui ou celle causé par une lésion ischémique de reperfusion du myocarde en circulation extracorporelle avec un coeur-poumon artificiel, ou celui ou celle causé par une opération à coeur ouvert comme un pontage aorto-coronaire sans utiliser un coeur-poumon artificiel.

5. Agent prophylactique ou thérapeutique pour utilisation selon l'une quelconque des revendications 1 à 4, le composé étant du (2S,4S)-6-fluoro-2',5'dioxospiro[chroman-4,4'-imidazolidine]-2-carboxamide (dénomination commune : fidarestat).
